# FASCICULE DE BREVET EUROPEEN

(11) **EP 3 727 534 B1**
(45) Date de publication et mention de la délivrance du brevet: **06.10.2021**
(21) Numéro de dépôt: 18833958.4
(22) Date de dépôt: 18.12.2018
(51) Int. Cl.: A61M 5/28, A61M 5/30, A61M 15/08, A61M 5/31, A61M 15/00, A61M 5/315, A61M 11/00

(54) **DISPOSITIF DE DISTRIBUTION DE PRODUIT FLUIDE**
VORRICHTUNG ZUR AUSGABE EINES FLUIDPRODUKTS
DEVICE FOR DISPENSING A FLUID PRODUCT

(30) Priorité: 22.12.2017 FR 1762963
(43) Date de publication de la demande: 28.10.2020
(73) Titulaire: APTAR France SAS, 27110 Le Neubourg (FR)
(72) Inventeur: PETIT, Ludovic, 27110 Vitot (FR)
(74) Mandataire: CAPRI
(86) Numéro de dépôt international: PCT/FR2018/053354
(87) Numéro de publication internationale: WO 2019/122671

(56) Documents cités:
- WO-A1-00/71262
- WO-A1-2016/097603
- FR-A1- 2 775 963

## Description

La présente invention concerne un dispositif de distribution de produit fluide.

Plus particulièrement, la présente invention concerne un dispositif de distribution de produit fluide pour distribuer un produit fluide pharmaceutique à un utilisateur, par exemple par l'intermédiaire d'une pulvérisation nasale. La présente invention pourrait toutefois aussi s'appliquer à des dispositifs de distribution de parfum ou de produits cosmétiques.

Dans ce type de dispositif, lors de l'actionnement, une aiguille vient généralement percer un bouchon-piston. Cette mise en œuvre présente notamment des risques de contamination du produit fluide par des particules du bouchon, libérées lors du perçage. Ces particules peuvent aussi dans certains cas occasionner un bouchage, partiel ou total, du conduit menant du réservoir à l'orifice de distribution, avec donc un risque de dysfonctionnement du dispositif de distribution. L'assemblage de l'aiguille de perçage, notamment son orientation et sa tenue dans le dispositif, peut aussi s'avérer complexe, avec par conséquent des risques de dysfonctionnement du dispositif.

Les documents FR2775963, WO0071262 et WO2016097603 décrivent des dispositifs de l'état de la technique.

La présente invention a pour but de fournir un dispositif de distribution de produit fluide qui ne reproduit pas les inconvénients susmentionnés.

Plus particulièrement, la présente invention a pour but de fournir un dispositif de distribution de produit fluide qui évite ou limite les risques de contamination du produit fluide.

La présente invention a également pour but de fournir un tel dispositif qui évite ou limite les risques de dysfonctionnement du dispositif.

La présente invention a également pour but de fournir un tel dispositif qui soit simple et peu coûteux à fabriquer et à assembler.

La présente invention a donc pour objet un dispositif de distribution de produit fluide, comportant un réservoir contenant du produit fluide, une tête de distribution pourvue d'un orifice de distribution, un piston pour distribuer au moins une partie dudit produit fluide à travers ledit orifice de distribution, ledit réservoir étant, avant actionnement, obturé de manière étanche par un élément obturateur, ledit dispositif comportant des moyens de passage pour relier ledit réservoir audit orifice de distribution lors de l'actionnement, lesdits moyens de passage comportant un manchon creux fixe par rapport à ladite tête de distribution, destiné à coulisser autour dudit élément obturateur lors de l'actionnement pour ouvrir lesdits moyens de passage.

Selon une première variante avantageuse, un joint radial est assemblé sur ledit manchon creux pour former un piston du dispositif, qui coopère de manière étanche avec ledit réservoir lors de l'actionnement pour refouler le produit fluide en direction dudit orifice de distribution.

Selon une seconde variante avantageuse, ledit manchon creux est formé d'une seule pièce avec une lèvre formant piston, qui coopère de manière étanche avec ledit réservoir lors de l'actionnement pour refouler le produit fluide en direction dudit orifice de distribution.

Avantageusement, ladite lèvre est formée au niveau d'un bord axial proximal dudit manchon creux.

Avantageusement, ledit manchon creux et ladite lèvre sont formés d'une seule pièce monobloc avec un organe creux disposé dans ladite tête de distribution, en amont dudit orifice de distribution, ledit organe creux définissant un canal d'expulsion.

En variante, ledit manchon creux est avantageusement assemblé dans un organe creux disposé dans ladite tête de distribution, en amont dudit orifice de distribution, ledit organe creux définissant un canal d'expulsion.

Avantageusement, ledit organe creux définit un profil de pulvérisation directement en amont dudit orifice de distribution.

Avantageusement, ledit manchon creux comporte un profil interne, tel qu'une ou plusieurs rainure(s) ou nervure(s), de sorte que lorsque ledit manchon creux est disposé autour dudit élément obturateur, ledit profil interne crée un ou plusieurs passage(s) de fluide autour dudit élément obturateur pour ouvrir lesdits moyens de passage.

En variante, ledit manchon creux comporte avantageusement un profil externe, tel qu'une ou plusieurs rainure(s) ou nervure(s), dans sa paroi cylindrique externe, de sorte que lorsque ledit manchon creux est disposé autour dudit élément obturateur, ledit profil externe crée un ou plusieurs passage(s) de fluide autour dudit élément obturateur, entre l'extérieur dudit manchon creux et une paroi interne dudit réservoir, pour ouvrir lesdits moyens de passage.

Avantageusement, ledit manchon creux comporte un canal radial pour ramener le liquide vers ledit canal d'expulsion, avec ledit piston disposé axialement de manière proximale par rapport audit canal radial.

Avantageusement, ledit élément obturateur est une bille.

En variante, ledit élément obturateur est un bouchon cylindrique.

Avantageusement, ledit élément obturateur est réalisé en matériau élastomère ou thermoplastique.

Selon un premier mode de réalisation avantageux, ledit réservoir contient une seule dose de produit fluide, distribuée en un seul actionnement du dispositif.

Selon un second mode de réalisation avantageux, ledit réservoir contient deux doses de produit fluide, distribuées en deux actionnements successifs du dispositif.

Ces caractéristiques et avantages et d'autres de la présente invention apparaitront plus clairement au cours de la description détaillée suivante, faite en référence aux dessins joints, donnés à titre d'exemples non limitatifs, et sur lesquels:
La figure 1 est une vue schématique en section transversale d'un dispositif de distribution de produit fluide selon un premier mode de réalisation avantageux, en position de repos avant actionnement,
La figure 2 est une vue similaire à celle de la figure 1, montrant le dispositif en début d'actionnement,
La figure 3 est une vue similaire à celles des figures 1 et 2, montrant le dispositif en fin d'actionnement,
La figure 4 est une vue similaire à celle de la figure 3, montrant une première variante de réalisation de l'élément obturateur,
La figure 5 est une vue similaire à celle de la figure 1, montrant une seconde variante de réalisation de l'élément obturateur,
La figure 6a est une vue de détail en section transversale d'une variante de réalisation du gicleur des figures 1 à 5,
La figure 6b est une vue agrandie du détail A de la figure 6a,
La figure 7 est une vue schématique en section transversale d'un dispositif de distribution de produit fluide selon un second mode de réalisation avantageux de la présente invention, en position de repos avant actionnement,
La figure 8a est une vue de détail en perspective découpée d'une variante de réalisation avantageuse du manchon creux, et
La figure 8b est une vue de détail agrandie d'une autre variante de réalisation avantageuse du manchon creux.

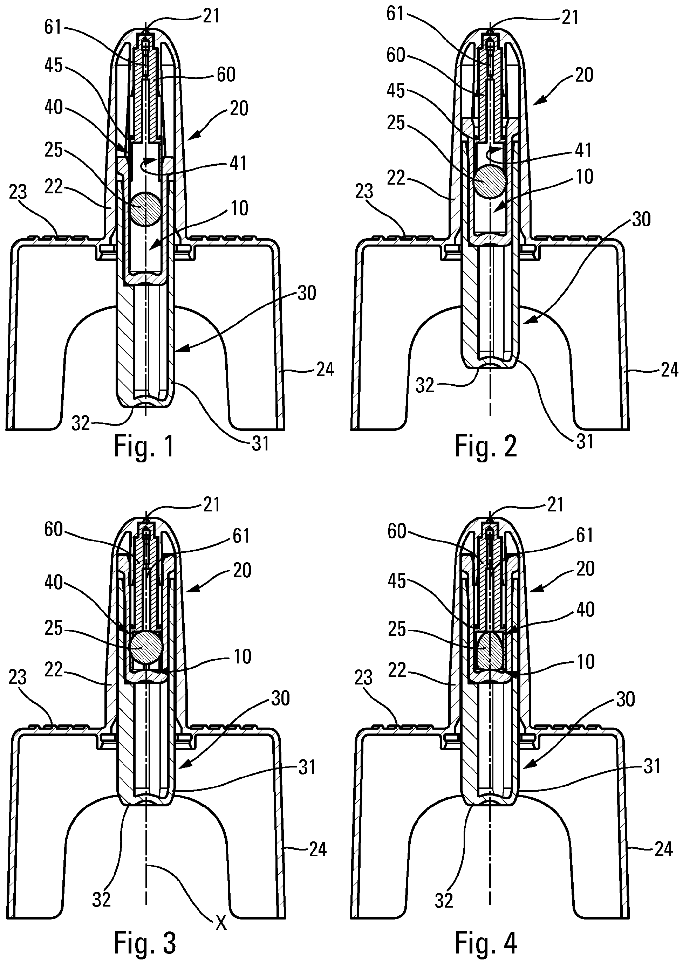

Les termes "proximal" et "distal" se réfèrent à l'orifice de distribution. Les termes "axial" et "radial" se réfèrent à l'axe central longitudinal X représenté notamment sur la figure 3. Les termes "amont" et "aval" se réfèrent à la direction d'écoulement du fluide lors de l'actionnement.

En référence aux figures 1 à 5, il est représenté plusieurs variantes d'un premier mode de réalisation avantageux de la présente invention.

Dans ce premier mode de réalisation, un réservoir 10 contenant du produit fluide à distribuer, typiquement un liquide, est disposé à l'intérieur d'un corps formant une tête de distribution 20. Cette tête de distribution 20 comporte un orifice de distribution 21, qui dans l'exemple des figures, est orienté axialement, mais qui pourrait être orienté différemment, par exemple radialement. Cet orifice de distribution 21 sert à distribuer une dose de produit fluide hors de ladite tête de distribution 20 lors de l'actionnement du dispositif par un utilisateur.

La tête de distribution 20 comporte avantageusement un embout nasal 22 comportant à son extrémité axiale proximale ledit orifice de distribution 21, ainsi qu'un corps latéral 24, relié audit embout nasal 22 par une bride radiale 23.

Avantageusement, un organe creux 60 est disposé dans la tête de distribution 20, en amont dudit orifice de distribution 21, ledit organe creux 60 définissant un canal d'expulsion 61. Avantageusement, ledit organe creux 60 peut aussi définir un profil de pulvérisation directement en amont dudit orifice de distribution 21.

Le réservoir 10 est dans les exemples des figures formé par un corps creux et borgne, comportant une seule ouverture fermée par un élément obturateur 25 et contenant une seule dose de produit fluide à distribuer en actionnement unique du dispositif.

L'élément obturateur 25 peut être une bille, comme illustré sur les figures 1 à 3 et 8a. Cette bille pourrait être en métal, mais avantageusement elle est réalisée en matériau élastomère ou thermoplastique, par exemple en COC (copolymère d'oléfine cyclique) ou en COC élastomère.

La figure 4 montre une variante dans laquelle l'élément obturateur 25 a une forme modifiée dans sa partie distale afin de limiter le volume mort dans le réservoir 10 en fin d'actionnement.

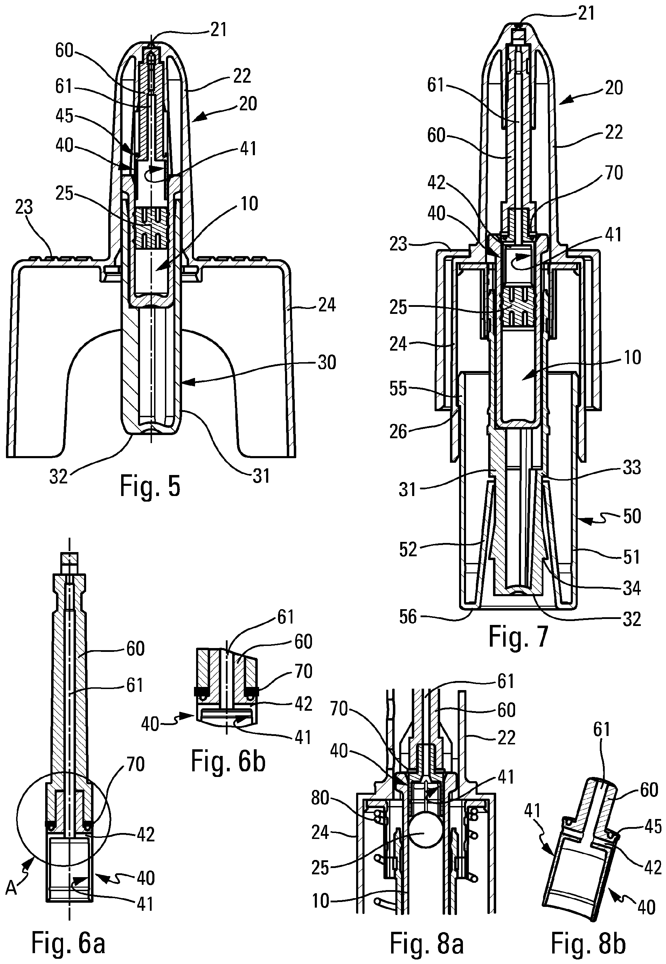

Les figures 5 et 7 illustrent une autre variante de réalisation, dans laquelle l'élément obturateur 25 forme un bouchon cylindrique similaire à un piston de seringue.

Il est à noter que la présente invention peut aussi s'adapter à des dispositifs du type bidose, contenant deux doses de produit fluide à distribuer en deux actionnements successifs du dispositif, comme représenté sur la figure 7.

Des moyens d'actionnement 30 sont prévus pour permettre l'actionnement du dispositif.

Dans les variantes des figures, ces moyens d'actionnement 30 comportent un corps d'actionnement 31 mobile par rapport à la tête de distribution 20, ledit corps d'actionnement 31 coopérant avec ledit réservoir 10 pour le déplacer axialement par rapport à la tête de distribution 20, en direction de l'orifice de distribution 21.

La tête de distribution 20 comporte des moyens de passage 40 qui relient, en cours d'actionnement, le réservoir 10 à l'orifice de distribution 21.

Selon l'invention, ces moyens de passage 40 comportent un manchon creux 41 fixe par rapport à la tête de distribution 20, et donc fixe par rapport à l'orifice de distribution 21, et qui est destiné à coopérer avec ledit élément obturateur 25 lors de l'actionnement pour ouvrir lesdits moyens de passage. Comme visible sur les figures, lors de l'actionnement, ledit manchon creux 41 vient coulisser autour dudit élément obturateur 25.

Ledit manchon creux 41 peut être formé d'une seule pièce avec une lèvre 45 formant piston, qui coopère de manière étanche avec le réservoir 10 lors de l'actionnement pour refouler le produit fluide en direction de l'orifice de distribution 21. Les figures 1 à 5 et 8b illustrent une telle variante. Dans ce cas, la lèvre 45 est formée au niveau du bord axial proximal dudit manchon creux 41. Avantageusement, ledit manchon creux 41 et ladite lèvre 45 peuvent être formés d'une seule pièce monobloc avec ledit organe creux 60, comme illustré sur les figures 1 à 5.

En variante, un joint radial 70 peut être assemblé sur ledit manchon creux 41, comme visible sur les figures 6a, 6b, 7 et 8a, pour former le piston du dispositif.

En variante, le manchon creux 41 et le piston, à savoir la lèvre 45 ou le joint radial 70, sont assemblés dans ledit organe creux 60, comme illustré sur les figures 6a à 8b

Dans une première variante de réalisation avantageuse, représentée sur les figures 1 à 5 et 8a, ledit manchon creux 41 comporte un profil interne, tel qu'une ou plusieurs rainure(s) ou nervure(s), de sorte que lorsque ledit manchon creux 41 est disposé autour dudit élément obturateur 25, ce profil interne crée un ou plusieurs passage(s) de fluide autour dudit élément obturateur 25 pour ouvrir les moyens de passage 40.

Dans une seconde variante de réalisation avantageuse, représentée sur les figures 6a, 6b, 7 et 8b, ledit manchon creux 41 comporte un profil externe, tel qu'une ou plusieurs rainure(s) ou nervure(s), dans sa paroi cylindrique externe, de sorte que lorsque ledit manchon creux 41 est disposé autour dudit élément obturateur 25, ce profil externe crée un ou plusieurs passage(s) de fluide autour dudit élément obturateur 25, entre l'extérieur dudit manchon creux 41 et la paroi interne du réservoir 10, pour ouvrir les moyens de passage 40. Dans ce cas, un canal radial 42 est prévu pour ramener le liquide vers le canal d'expulsion 61, avec le piston 45, 70 disposé axialement de manière proximale par rapport audit canal radial 42.

Le fonctionnement du dispositif va être détaillé ci-après.

De manière classique, dans les exemples représentés sur les figures, l'utilisateur place deux doigts sur la bride radiale 23 formée sur la tête de distribution 20, et appuie avec le pouce sur le fond axial distal 32 dudit corps d'actionnement 31. Lors d'un tel actionnement, le réservoir 10 est donc poussé axialement en direction de l'orifice de distribution 21, de sorte que le manchon creux 41 va coulisser autour de l'élément obturateur 25. Le contenu du réservoir 10 est alors relié à l'orifice de distribution 21 et l'appui de l'utilisateur sur le corps d'actionnement 31 déplace le piston 45, 70 dans le réservoir 10 pour assurer la distribution du produit fluide.

Dans un autre mode de réalisation, non représenté sur les dessins, le réservoir pourrait ne pas être formé par un corps creux borgne ne comportant qu'une ouverture, mais par un cylindre creux ouvert axialement des deux côtés. Ce cylindre serait alors fermé du côté proximal par un premier élément obturateur et du côté distal par un second élément obturateur, le volume défini entre lesdits deux éléments obturateurs contenant le produit fluide à distribuer. Lorsque l'utilisateur actionne le dispositif, il va comme décrit précédemment appuyer axialement sur le corps d'actionnement pour le faire coulisser axialement vers l'orifice de distribution. Ceci provoque le déplacement du second élément obturateur à l'intérieur du réservoir. Or, le produit fluide étant incompressible, ce déplacement du second élément obturateur va donc déplacer le premier élément obturateur en direction du manchon creux, qui est fixe. Le premier élément obturateur va donc être entouré par ledit manchon creux et le contenu du réservoir va être distribué à travers le passage formé soit entre ledit manchon creux et ledit premier élément obturateur, soit entre ledit manchon creux et ledit réservoir, avec le second élément obturateur qui agit alors en tant que piston.

Comme évoqué précédemment, l'invention peut aussi s'appliquer à un dispositif du type bidose, tel que représenté sur la figure 7, formant un second mode de réalisation. Dans ce cas, le contenu du réservoir serait distribué en deux actionnements successifs. Le document WO02014147329 décrit un exemple de dispositif bidose.

Dans l'exemple de la figure 7, un corps externe 50 est assemblé autour du corps d'actionnement 31, ledit corps externe 50 comportant un manchon cylindrique 51 et au moins une patte inclinée 52 qui est adaptée à coopérer avec des projections 33, 34 du corps d'actionnement 31 pour réaliser les actionnements successifs. L'utilisateur appuie alors de préférence sur le bord axial inférieur 56 dudit corps externe pour réaliser l'actionnement du dispositif. Ledit corps externe 50 est avantageusement retenu axialement sur ladite tête de distribution 20, par coopération de l'extrémité axiale proximale 55 dudit manchon cylindrique 51 sur un épaulement 26 prévu dans ledit corps latéral 24 de ladite tête de distribution 20. Un ressort de rappel 80, visible sur la figure 8a, est monté entre le corps externe 50 et la tête de distribution 20 pour ramener ledit corps externe 50 dans sa position de départ après chaque actionnement.

Bien entendu, d'autres variantes de réalisation sont également envisageables, sans sortir du cadre de la présente invention, tel que défini par les revendications annexées.

## Revendications

1. Dispositif de distribution de produit fluide, comportant un réservoir (10) contenant du produit fluide, une tête de distribution (20) pourvue d'un orifice de distribution (21), un piston (45 ; 70) pour distribuer au moins une partie dudit produit fluide à travers ledit orifice de distribution (21), ledit réservoir (10) étant, avant actionnement, obturé de manière étanche par un élément obturateur (25), ledit dispositif comportant des moyens de passage (40) pour relier ledit réservoir (10) audit orifice de distribution (21) lors de l'actionnement, **caractérisé en ce que** lesdits moyens de passage (40) comportent un manchon creux (41) fixe par rapport à ladite tête de distribution (20), destiné à coulisser autour dudit élément obturateur (25) lors de l'actionnement pour ouvrir lesdits moyens de passage (40).

2. Dispositif selon la revendication 1, dans lequel un joint radial (70) est assemblé sur ledit manchon creux (41) pour former un piston du dispositif, qui coopère de manière étanche avec ledit réservoir (10) lors de l'actionnement pour refouler le produit fluide en direction dudit orifice de distribution (21).

3. Dispositif selon la revendication 1, dans lequel ledit manchon creux (41) est formé d'une seule pièce avec une lèvre (45) formant piston, qui coopère de manière étanche avec ledit réservoir (10) lors de l'actionnement pour refouler le produit fluide en direction dudit orifice de distribution (21).

4. Dispositif selon la revendication 3, dans lequel ladite lèvre (45) est formée au niveau d'un bord axial proximal dudit manchon creux (41).

5. Dispositif selon la revendication 3 ou 4, dans lequel ledit manchon creux (41) et ladite lèvre (45) sont formés d'une seule pièce monobloc avec un organe creux (60) disposé dans ladite tête de distribution (20), en amont dudit orifice de distribution (21), ledit organe creux (60) définissant un canal d'expulsion (61).

6. Dispositif selon l'une quelconque des revendications 1 à 4, dans lequel ledit manchon creux (41) est assemblé dans un organe creux (60) disposé dans ladite tête de distribution (20), en amont dudit orifice de distribution (21), ledit organe creux (60) définissant un canal d'expulsion (61).

7. Dispositif selon la revendication 5 ou 6, dans lequel ledit organe creux (60) définit un profil de pulvérisation directement en amont dudit orifice de distribution (21).

8. Dispositif selon l'une quelconque des revendications précédentes, dans lequel ledit manchon creux (41) comporte un profil interne, tel qu'une ou plusieurs rainure(s) ou nervure(s), de sorte que lorsque ledit manchon creux (41) est disposé autour dudit élément obturateur (25), ledit profil interne crée un ou plusieurs passage(s) de fluide autour dudit élément obturateur (25) pour ouvrir lesdits moyens de passage (40).

9. Dispositif selon l'une quelconque des revendications 1 à 7, dans lequel ledit manchon creux (41) comporte un profil externe, tel qu'une ou plusieurs rainure(s) ou nervure(s), dans sa paroi cylindrique externe, de sorte que lorsque ledit manchon creux (41) est disposé autour dudit élément obturateur (25), ledit profil externe crée un ou plusieurs passage(s) de fluide autour dudit élément obturateur (25), entre l'extérieur dudit manchon creux (41) et une paroi interne dudit réservoir (10), pour ouvrir lesdits moyens de passage (40).

10. Dispositif selon la revendication 9 lorsque dépendante de l'une quelconque des revendications 5 à 7, dans lequel ledit manchon creux (41) comporte un canal radial (42) pour ramener le liquide vers ledit canal d'expulsion (61), avec ledit piston (45; 70) disposé axialement de manière proximale par rapport audit canal radial (42).

11. Dispositif selon l'une quelconque des revendications précédentes, dans lequel ledit élément obturateur (25) est une bille.

12. Dispositif selon l'une quelconque des revendications 1 à 10, dans lequel ledit élément obturateur (25) est un bouchon cylindrique.

13. Dispositif selon l'une quelconque des revendications précédentes, dans lequel ledit élément obturateur (25) est réalisé en matériau élastomère ou thermoplastique.

14. Dispositif selon l'une quelconque des revendications précédentes, dans lequel ledit réservoir (10) contient une seule dose de produit fluide, distribuée en un seul actionnement du dispositif.

15. Dispositif selon l'une quelconque des revendications 1 à 13, dans lequel ledit réservoir (10) contient deux doses de produit fluide, distribuées en deux actionnements successifs du dispositif.

## Patentansprüche

1. Vorrichtung zur Abgabe eines flüssigen Produkts, umfassend einen Vorratsbehälter (10) mit einer darin enthaltenen Flüssigkeit, einen mit einer Abgabeöffnung (21) versehenen Abgabekopf (20), einen Kolben (45; 70) zur wenigstens teilweisen Abgabe des flüssigen Produkts über die Abgabeöffnung (21), wobei vor Betätigung der Vorratsbehälter (10) durch ein Verschlusselement (25) dicht verschlossen ist, wobei die Vorrichtung Durchlasseinrichtungen (40) umfasst, um bei Betätigung den Vorratsbehälter (10) mit der Abgabeöffnung (21) zu verbinden, **dadurch gekennzeichnet, dass** die Durchlasseinrichtungen (40) eine hohle Hülse (41) umfassen, die relativ zum Abgabekopf (20) angebracht ist und die bei Betätigung um das Verschlusselement (25) herum verschieblich ist, um die Durchlasseinrichtungen (40) zu öffnen.

2. Vorrichtung nach Anspruch 1, bei der eine Radialdichtung (70) an der hohlen Hülse (41) angebracht ist, um so einen Kolben der Vorrichtung zu bilden, welcher bei Betätigung abdichtend mit dem Vorratsbehälter (10) zusammenwirkt, um das flüssige Produkt zur Abgabeöffnung (21) hin zu verdrängen.

3. Vorrichtung nach Anspruch 1, bei der die hohle Hülse (41) einstückig mit einer Kolbenlippe (45) ausgebildet ist, welche bei Betätigung abdichtend mit dem Vorratsbehälter (10) zusammenwirkt, um das flüssige Produkt zur Abgabeöffnung (21) hin zu verdrängen.

4. Vorrichtung nach Anspruch 3, bei der die Lippe (45) an einer proximalen axialen Kante der hohlen Hülse (41) ausgebildet ist.

5. Vorrichtung nach Anspruch 3 oder 4, bei der die hohle Hülse (41) und die Lippe (45) einstückig mit einem Hohlkörper (60) ausgebildet sind, der in dem Abgabekopf (20) stromaufwärts der Abgabeöffnung (21) angeordnet ist, wobei der Hohlkörper (60) einen Ausstoßkanal (61) definiert.

6. Vorrichtung nach einem der Ansprüche 1 bis 4, bei der die hohle Hülse (41) in einen Hohlkörper (60) eingebaut ist, der in dem Abgabekopf (20) stromaufwärts von der Abgabeöffnung (21) angeordnet ist, wobei der Hohlkörper (60) einen Ausstoßkanal (61) definiert.

7. Vorrichtung nach Anspruch 5 oder 6, bei dem das Hohlelement (60) ein Zerstäubungsprofil unmittelbar stromaufwärts von der Abgabeöffnung (21) definiert.

8. Vorrichtung nach einem der vorhergehenden Ansprüche, bei der die hohle Hülse (41) ein inneres Profil, beispielsweise eine oder mehrere Rille(n) oder Rippe(n), aufweist, sodass, wenn die hohle Hülse (41) um das Verschlusselement (25) herum angeordnet ist, durch das innere Profil ein bzw. mehrere Flüssigkeitsdurchlass bzw. -lässe um das Verschlusselement (25) herum gebildet wird bzw. werden, um die Durchlasseinrichtungen (40) zu öffnen.

9. Vorrichtung nach einem der Ansprüche 1 bis 7, bei der die hohle Hülse (41) ein äußeres Profil, beispielsweise eine oder mehrere Rille(n) oder Rippe(n), in ihrer äußeren zylindrischen Wand aufweist, sodass, wenn die hohle Hülse (41) um das Verschlusselement (25) herum angeordnet ist, durch das äußere Profil ein bzw. mehrere Flüssigkeitsdurchlass bzw. -lässe um das Verschlusselement (25) herum, zwischen der Außenseite der hohlen Hülse (41) und einer Innenwand des Vorratsbehälters (10), gebildet wird bzw. werden, um die Durchlasseinrichtungen (40) zu öffnen.

10. Vorrichtung nach Anspruch 9 in Abhängigkeit von einem der Ansprüche 5 bis 7, bei der die hohle Hülse (41) einen radialen Kanal (42) zur Rückführung der Flüssigkeit in den Ausstoßkanal (61) aufweist, wobei der Kolben (45; 70) axial proximal zu dem radialen Kanal (42) angeordnet ist.

11. Vorrichtung nach einem der vorhergehenden Ansprüche, bei der das Verschlusselement (25) eine Kugel ist.

12. Vorrichtung nach einem der Ansprüche 1 bis 10, bei der das Verschlusselement (25) ein zylindrischer Stopfen ist.

13. Vorrichtung nach einem der vorhergehenden Ansprüche, bei der das Verschlusselement (25) aus elastomerem oder thermoplastischem Material hergestellt ist.

14. Vorrichtung nach einem der vorhergehenden Ansprüche, bei der der Vorratsbehälter (10) eine Einzeldosis eines flüssigen Produkts enthält, deren Abgabe bei einmaliger Betätigung der Vorrichtung erfolgt.

15. Vorrichtung nach einem der Ansprüche 1 bis 13, bei der der Vorratsbehälter (10) zwei Dosen eines flüssigen Produkts enthält, deren Abgabe bei zweimal hintereinander durchgeführter Betätigung der Vorrichtung erfolgt.

## Claims

1. A fluid dispenser device comprising: a reservoir (10) containing fluid; a dispenser head (20) that is provided with a dispenser orifice (21), a piston (45; 70) for dispensing at least a portion of said fluid through said dispenser orifice (21), said reservoir (10) being closed in leaktight manner by a closure element (25) before actuation, said device including passage means (40) for connecting said reservoir (10) to said dispenser orifice (21) during actuation, the device being **characterized in that** said passage means (40) comprise a hollow sleeve (41) that is stationary relative to said dispenser head (20), for sliding around said closure element (25) during actuation, so as to open said passage means (40).

2. A device according to claim 1, wherein a radial gasket (70) is assembled on said hollow sleeve (41) so as to form a piston of the device, which piston co-operates in leaktight manner with said reservoir (10) during actuation, so as to drive the fluid towards said dispenser orifice (21).

3. A device according to claim 1, wherein said hollow sleeve (41) is formed integrally with a piston-forming lip (45) that co-operates in leaktight manner with said reservoir (10) during actuation, so as to drive the fluid towards said dispenser orifice (21).

4. A device according to claim 3, wherein said lip (45) is formed at a proximal axial edge of said hollow sleeve (41).

5. A device according to claim 3 or claim 4, wherein said hollow sleeve (41) and said lip (45) are formed integrally with a hollow member (60) that is arranged in said dispenser head (20), upstream from said dispenser orifice (21), said hollow member (60) defining an expulsion channel (61).

6. A device according to any one of claims 1 to 4, wherein said hollow sleeve (41) is assembled in a hollow member (60) that is arranged in said dispenser head (20), upstream from said dispenser orifice (21), said hollow member (60) defining an expulsion channel (61).

7. A device according to claim 5 or claim 6, wherein said hollow member (60) defines a spray profile directly upstream from said dispenser orifice (21).

8. A device according to any preceding claim, wherein said hollow sleeve (41) includes an inner profile, such as one or more grooves or splines, so that when said hollow sleeve (41) is arranged around said closure element (25), said inner profile creates one or more fluid passages around said closure element (25), so as to open said passage means (40).

9. A device according to any one of claims 1 to 7, wherein the outer cylindrical wall of said hollow sleeve (41) has an outer profile, such as one or more grooves or splines, so that when said hollow sleeve (41) is arranged around said closure element (25), said outer profile creates one or more fluid passages around said closure element (25), between the outside of said hollow sleeve (41) and an inner wall of said reservoir (10), so as to open said passage means (40).

10. A device according to claim 9, when dependent on any one of claims 5 to 7, wherein said hollow sleeve (41) includes a radial channel (42) for guiding the liquid towards said expulsion channel (61), with said piston (45, 70) arranged axially in proximal manner relative to said radial channel (42).

11. A device according to any preceding claim, wherein said closure element (25) is a ball.

12. A device according to any one of claims 1 to 10, wherein said closure element (25) is a cylindrical stopper.

13. A device according to any preceding claim, wherein said closure element (25) is made out of elastomer or thermoplastic material.

14. A device according to any preceding claim, wherein said reservoir (10) contains a single dose of fluid, for dispensing during a single actuation of the device.

15. A device according to any one of claims 1 to 13, wherein said reservoir (10) contains two doses of fluid, for dispensing during two successive actuations of the device.
